# EUROPEAN PATENT APPLICATION

(11) **EP 1 795 151 A1**
(43) Date of publication of application: **13.06.2007**
(21) Application number: 05026668.3
(22) Date of filing: 07.12.2005
(51) Int. Cl.: A61F 2/06

(54) **Biodegradable stent**

(71) Applicant: Dr. Karel Volenec - ELLA - CS, 500 06 Hradec Kralove (CZ)
(72) Inventor: Volenec Karel, Hradec Kralove (CZ); Kubena, Petr, Hradec Kralove (CZ); Danis Jan, Hainburg an der Donau (AT)

(57) **Abstract**

This invention relates to stents implanted into gastrointestinal tract.

The biodegradable stent is formed from one piece of the degradable fiber (1). The edges thereof are ended with non-traumatic bends (2). A left winding 3 and right winding 4 are interlaced together to create a regular mesh. The mesh of the body of the biodegradable stent includes non-shortening portions (5).

Both its flared ends are rounded inward at the very edges.

The free ends of the degradable fiber are connected together in the middle of the biodegradable stent body, where one radiopaque marker (6) is fastened. The other four radiopaque markers (6) are fastened at both biodegradable stent ends.

Both its ends are equipped with a string (7), circumferentially interlaced through the mesh.

The biodegradable stent is equipped with an anti-migration member (8), circumferentially fastened to its mesh in its proximal part.

## Description

This invention relates generally to medical devices. More particularly, the field of art into which this invention falls is stents implanted into tubular organs of the gastrointestinal tract of a human body.

Stents, i.e. medical devices securing patency of tubular organs, are commonly used in medical practice. If they are used for palliative treatment of malignant stenoses - i.e. the stent is not considered to be removed from the patient's body, no special demands are made thereon. However, benign stenoses, among others, also fall into a stent indication; in addition to this, stents are also used for overlapping a wound dehiscence in surgical anastomoses in gastrointestinal tract or stopping bleeding from esophageal varices. In such cases the stent is intended to be removed one day. If the stent is implanted in situ longer than one week, it is then so-called embedded or even ingrown in the tissue; its removal is connected with troubles and may even lead to a serious deterioration of the tissue.
The most appropriate solution is degradable stent the degradation of which is controlled in a due manner. Such a stent will not be removed because, once its functioning is finished or the reason for being implanted is over, it degrades and gradually passes out of the patient's body in a natural way; possibly, the final degradation products get absorbed.
Nowadays, fully degradable materials are at our disposal, however, stents made of them have a big disadvantage - they must be expanded by using a balloon. If such a stent is required to be self-expanding, it would have to be made of a degradable wire of a large diameter or of a degradable tube with a thick wall. The stents like this require a delivery system of a large diameter, which is in a stark contrast to clinical needs as far as the safety is concerned.

The submitted technical solution of the degradable stent is a compromise between a balloon-expandable and self-expanding stent - the biodegradable stent of the present invention is self-expanding and concurrently keeps the parameters, which allow use in a delivery system of a common diameter. This has been achieved by using an appropriate degradable material, e.g. absorbable polydioxanone monofilamentous fiber - either single or double, of which the biodegradable stent is made.
The biodegradable stent can be either non-covered or covered with a degradable covering, if needed.

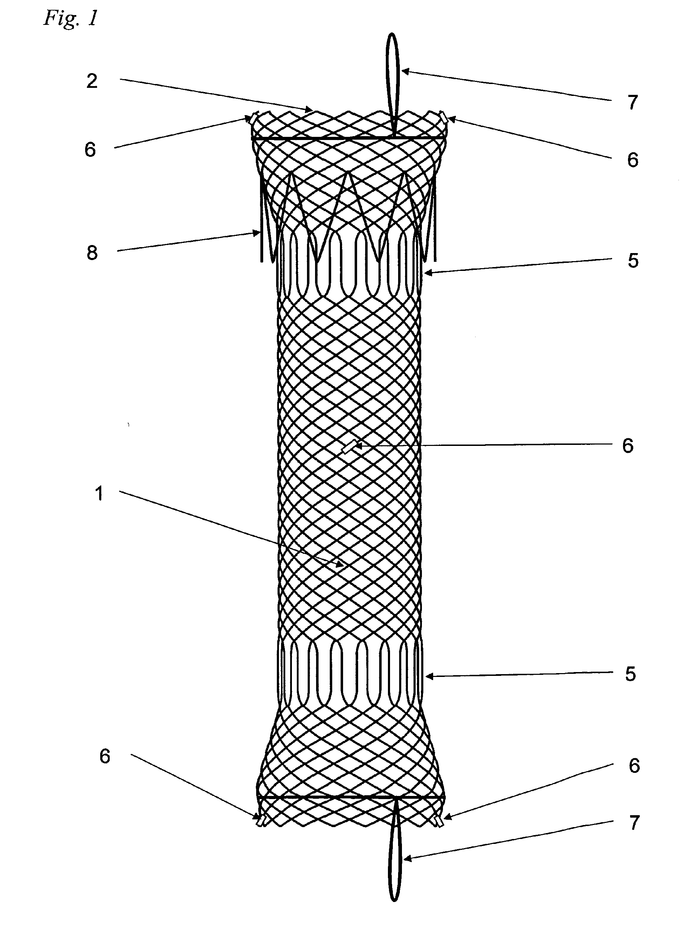

Fig. 1 is a side view of the preferred embodiment of the biodegradable stent.
Fig. 2 is an enlarged detail of the preferred embodiment of the biodegradable stent from the Fig. 1

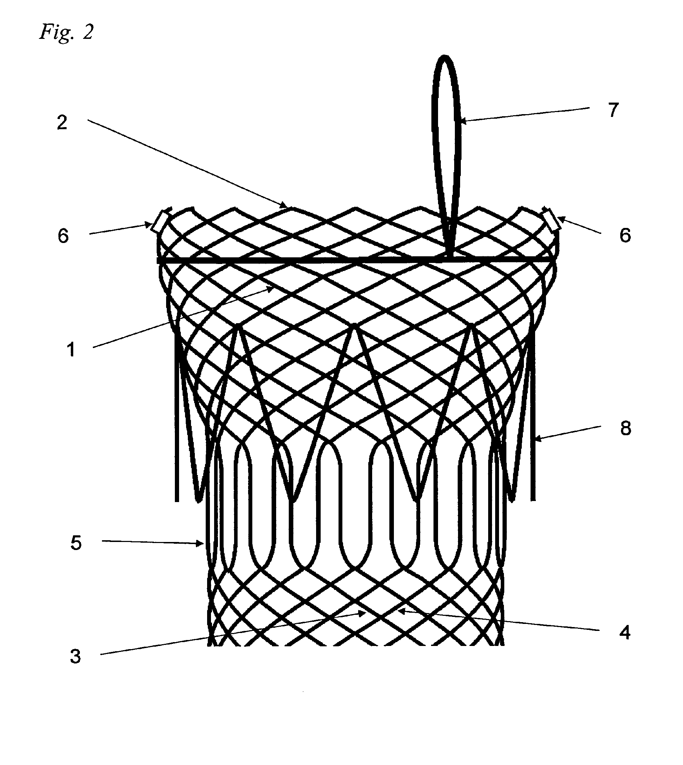

The degradable fiber 1 that a preferred embodiment of the biodegradable stent is formed from is one piece of the single absorbable polydioxanone monofilamentous fiber. The edges of the biodegradable stent ends are ended with non-traumatic bends 2 (as shown in the Fig. 2) not to injure the tissue. The method of braiding of the preferred embodiment of the biodegradable stent is based on left winding 3 and right winding 4 that are interlaced together to create a regular mesh. To lower shortening of the biodegradable stent, the mesh of the body thereof includes two non-shortening portions 5 located at ends, before flares. Said non-shortening portions 5 are formed such that left winding 3 is bent right and right winding 4 is bent left such that both windings lead lengthwise with the longitudinal axis of the biodegradable stent. Subsequently, the left winding 3 is bent left and right winding 4 is bent right, due to which a regular mesh is formed again.
Both biodegradable stent flared ends are rounded inward at the very edges to be more acceptable for a patient's tissue.
The free ends of the said fiber are connected together in the middle of the biodegradable stent body, in a place where one radiopaque marker 6 is fastened. The other four radiopaque markers 6 are fastened at both ends - two at proximal end, two at distal end, opposite each other.
Both proximal and distal flared end is equipped with a string 7, which is circumferentially interlaced through the mesh. Said string 7 serves for reducing of the diameter if being pulled.
The preferred embodiment of the biodegradable stent is equipped with an anti-migration member 8. It is also made of single absorbable polydioxanone monofilamentous fiber, bent in a zigzag shape and circumferentially fastened to the biodegradable stent mesh in its proximal part. The anti-migration member 8 is flexible in a proximal-distal direction, which allows a reposition of the released biodegradable stent if misplaced.
When being implanted, the biodegradable stent is introduced to the desired place of a tubular organ by means of a delivery system, inside which the biodegradable stent is compressed lengthwise. The biodegradable stent is subsequently released therefrom and radially expands. The delivery system is then removed from the patient's body.

Due to an influence of the tissue and food in the gastrointestinal tract, the structure of the polydioxanone monofilamentous fiber turns disrupted and gradually degrades after some time. The final products of the degradation are water and carbon dioxide. The degradation results in disintegration of the biodegradable stent and its degraded parts leave the patient's body in a natural way. The said final products get also partly absorbed by the patient's tissue to some extent.

## Claims

1. A biodegradable stent designed to secure patency of tubular organs, braided from one piece of degradable fiber, comprising a tubular-shaped, self-expanding structure based on left winding (3) and right winding (4) that are interlaced together to create a regular mesh, **characterized in that** said structure includes two non-shortening portions (5) located at ends, before flares, which are formed such that left winding (3) is bent right and right winding (4) is bent left such that both windings lead lengthwise with the longitudinal axis of the biodegradable stent, then the left winding (3) is bent left and right winding (4) is bent right, due to which a regular mesh is formed again.

2. The biodegradable stent according to Claim 1, **characterized in that** it is braided from single degradable fiber (1) or double degradable fiber.

3. The biodegradable stent according to Claim 1, **characterized in that** the degradable fiber (1), of which it is braided from, is absorbable polydioxanone monofilamentous fiber allowing a control of the degradation by means of used diameter thereof.

4. The biodegradable stent according to Claim 1, **characterized in that** the free ends of the said degradable fiber (1) are connected together in the middle of the stent body, which causes the stent edges to be ended with non-traumatic bends (2), in a place where one radiopaque marker (6) is fastened, whereas the other four radiopaque markers (6) are fastened at both ends - two at proximal end, two at distal end, opposite each other.

5. The biodegradable stent according to Claim 1, **characterized in that** it is equipped with an anti-migration member (8), which is also made of single absorbable polydioxanone monofilamentous fiber, circumferentially fastened to the biodegradable stent mesh in its proximal part such that it is flexible in a proximal-distal direction, which allows a reposition of the released biodegradable stent.

6. The biodegradable stent according to Claim 1, **characterized in that** both proximal and distal end is equipped with a string (7) reducing a diameter thereof.
